# EUROPEAN PATENT APPLICATION

(11) **EP 2 907 398 A1**
(43) Date of publication of application: **19.08.2015**
(21) Application number: 14156264.5
(22) Date of filing: 21.02.2014
(51) Int. Cl.: A24F 47/00, A61M 11/04

(54) **Disposable electronic cigarette**

(30) Priority: 27.01.2014 CN 201420052600 U
(71) Applicant: Shenzhen Smaco Technology Limited, 518106 (CN)
(72) Inventor: Wu, Yangyang, Shenzhen 518106 (CN)
(74) Representative: Jeannet, Olivier

(57) **Abstract**

The invention discloses a disposable electronic cigarette, comprising a battery assembly (1) and an atomizer (2). The battery assembly (1) comprises a cigarette tubular housing (11) and a battery (12); the atomizer (2) comprises a casing (21), tobacco tar cotton (22), a U-shaped tar guiding rope (23), a hollow ejector pin (24), a first fiber tube (25), a heating wire (26), conducting wires and a second fiber tube (29). A connection thread (13) is arranged at an end opening where the battery assembly (1) is connected with the atomizer (2), the thread (13) is electrically connected with the negative electrode of the battery, and a battery positive electrode contact is arranged in the center of the end opening; the atomizer (2) is provided with a conductive screw sleeve (30) that fits the connection thread (13); one end of the ejector pin (24) is connected with the battery positive electrode contact; one end of the heating wire (26) is connected with the ejector pin (24) via the conducting wire (27) while the other end thereof is electrically connected with the conductive screw sleeve (30) via the other conducting wire (28). The disposable electronic cigarette of the invention is large in smoke volume, low in odor generating possibility, simple in structure, convenient for assembly and capable of improving productivity and lowering production cost remarkably.

## Description

### Technical Field

The invention relates to an electronic cigarette, in particular to a disposable electronic cigarette with large smoke volume and low odor generating possibility. The disposable electronic cigarette is simple in structure, convenient for assembly and capable of lowering production cost remarkably.

### Background Art

It is well known that smoking is harmful to health, nicotine and tar in cigarettes pose a severe threat to the body health of people, and legislative bans on smoking or ban smoking in public places has begun in many countries. Currently in these countries, however, there still are a large number of smokers, who smoke heavily, are highly dependent on cigarettes and fail to quit smoking within a short period of time. Therefore, healthy and environment-friendly electronic cigarettes have been gradually accepted by many smokers.

At present, electronic cigarettes that are commonly available on the market are composed of a battery assembly and an atomizer, and addiction of smokers is alleviated in such a manner that the battery assembly is electrified to heat tobacco tar to generate smoke. However, electronic cigarettes in the prior art have small smoke volume and poor tobacco oil flowability, tends to generate odor during smoking and affect the smoking feeling of smokers severely; furthermore, these electronic cigarettes are complex in structure, tedious in assembly process, low in production efficiency and extremely high in cost.

### Summary of the Invention

An object of the invention is to provide a disposable electronic cigarette with large smoke volume and low odor generating possibility, in order to overcome the shortcomings in the prior art. The disposable electronic cigarette is simple in structure, convenient for assembly and capable of improving productivity and lowering production cost remarkably.

To reach the object above, adopted in the invention is the technical solution below:
A disposable electronic cigarette comprises a battery assembly and an atomizer.
The battery assembly comprises,

A cigarette tubular housing, wherein one end of the housing is sealed while the other end thereof is provided with an end opening for fitting connection with the atomizer, the end opening is provided with positive and negative electrodes capable of electric connection with the atomizer, and a cavity with a battery therein is arranged inside the housing; and

A battery, wherein the battery is arranged inside the cavity of the housing, and the positive and negative electrodes of the battery are electrically connected with the positive and negative electrodes at the end opening of the housing;

The atomizer comprises,

A casing having an outer diameter matched with the housing of the battery assembly;

A cylindrical tobacco tar cotton arranged inside the casing, wherein the tobacco tar cotton is of a hollow structure;

A U-shaped tar guiding rope arranged inside the tobacco tar cotton, wherein the both sides of the U-shaped tar guiding rope come into full contact with the tobacco tar cotton, and the sealed end at the bottom is arranged in an airflow channel in the hollow tube of the tobacco tar cotton;

A hollow ejector pin, wherein one end of the hollow ejector pin is electrically connected with the corresponding electrode of the battery assembly and communicated with an air inlet, while the other end thereof is sleeved with a first fiber tube and arranged inside the hollow tube of the tobacco tar cotton, and an air outlet of the ejector pin faces towards the sealed end at the bottom of the tar guiding rope;

A heating wire, wherein the heating wire is wound on the sealed end at the bottom of the tar guiding rope in a noncontact manner, one end of the heating wire is connected with the ejector pin via a conducting wire, and the other end thereof is electrically connected with the other electrode of the battery assembly via the other conducting wire; and

A second fiber tube, wherein the second fiber tube is concentrically arranged at the other side of the heating wire corresponding to the air outlet of the ejector pin, forms the airflow channel together with the first fiber tube, and is arranged on the cigarette holder-neighboring end inside the tobacco tar cotton, in order to achieve isolation from tobacco tar.

Preferably, a connection thread is arranged at the end opening where the battery assembly is connected with the atomizer, the thread is electrically connected with the negative electrode of the battery, and a battery positive electrode contact is arranged in the center of the end opening;

The atomizer is provided with a conductive screw sleeve that fits the connection thread;

One end of the ejector pin is connected with the battery positive electrode contact;

One end of the heating wire is connected with the ejector pin via the conducting wire while the other end thereof is electrically connected with the conductive screw sleeve via the other conducting wire.

Preferably, a rubber cushion is arranged on the inner wall of the conductive screw sleeve and is made of a silica gel material, and the ejector pin is arranged inside the rubber cushion.

Preferably, a hollow rubber sheath for sealing and isolation is further arranged inside the cigarette holder-neighboring casing of the atomizer, and the rubber sheath is made of a silica gel material.

Preferably, an atomizer cover that fits the rubber sheath is arranged inside the cigarette holder-neighboring casing of the atomizer.

By adopting the above technical solution, the disposable electronic cigarette of the present invention is capable of generating a large amount of smoke when heat is generated by discharging of the atomizer because full contact is achieved between the tar guiding rope and the tobacco tar cotton; and the disposable electronic cigarette avoids such a situation that odor results from excessive temperature and tobacco oil decomposition because of lack of tobacco oil. Furthermore, the disposable electronic cigarette of the present invention is simple in structure, convenient for assembly and capable of improving production efficiency and lowering cost remarkably.

### Brief description of the drawings

Fig.1 is an exploded structure view of the present invention;
Fig.2 is a sectional view of the present invention along an axial direction;
Fig.3 is an enlarged view of A in Fig.2.

### Detailed Description of the Preferred Embodiments

The present invention will be further described below in details in conjunction with the accompanying drawings and the embodiments.

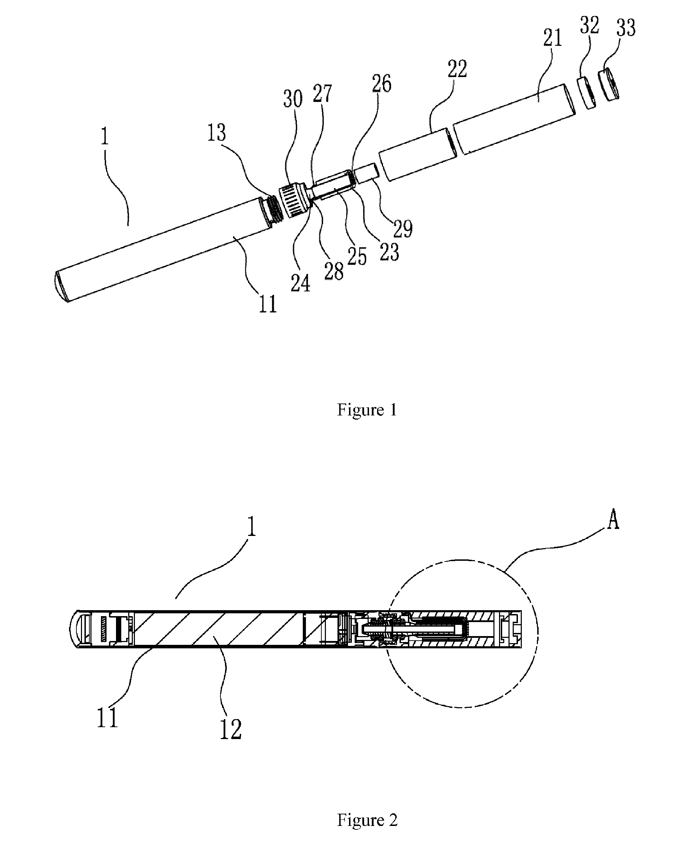

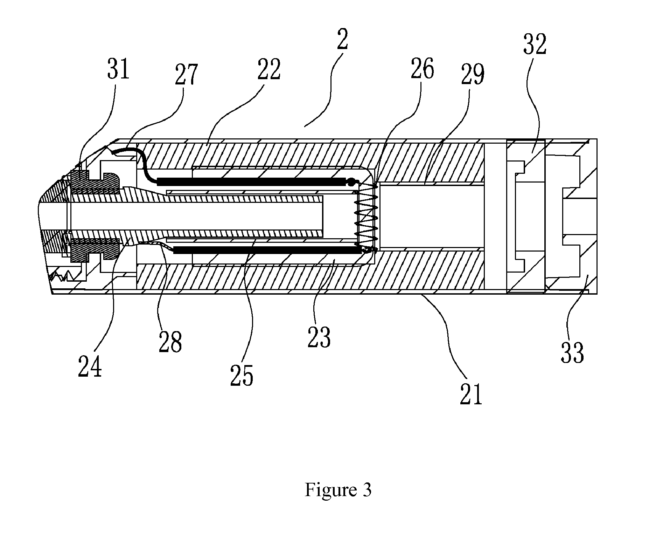

As shown in Fig.1 to Fig.3, a disposable electronic cigarette comprises a battery assembly 1 and an atomizer 2; the battery assembly 1 comprises a cigarette tubular housing 11 and a battery 12; one end of the housing 11 is sealed while the other end thereof is provided with an end opening for fitting connection with the atomizer 2, the end opening is provided with a connection thread 13, the thread 13 is electrically connected with the negative electrode of the battery 12, a battery positive electrode contact is arranged in the center of the end opening, and a cavity with the battery 12 therein is arranged inside the housing 11; and the battery 12 is arranged inside the cavity of the housing 11, and the positive and negative electrodes of the battery 12 are electrically connected with the positive and negative electrodes at the end opening of the housing respectively.

The atomizer 2 is provided with a conductive screw sleeve 30 that fits the connection thread 13, a rubber cushion 31 is arranged on the inner wall of the conductive screw sleeve 30, and the rubber cushion 31 is made of a silica gel material. The atomizer 2 comprises a casing 21, tobacco tar cotton 22, a U-shaped tar guiding rope 23, an ejector pin 24, a first fiber tube 25, a heating wire 26, a conducting wire 27, a conducting wire 28 and a second fiber tube 29; the U-shaped guiding rope is arranged inside the tobacco tar cotton 22, the both sides of the U-shaped tar guiding rope 23 come into full contact with the tobacco tar cotton 22, and the sealed end at the bottom is arranged in an airflow channel in the hollow tube of the tobacco tar cotton; the ejector pin 24 is arranged inside the rubber cushion 31, one end of the ejector pin 24 is electrically connected with the corresponding positive electrode of the battery assembly 1 and communicated with an air inlet while the other end thereof is sleeved with the first fiber tube 25 and arranged inside the hollow tube of the tobacco tar cotton 22, and an air outlet of the ejector pin faces towards the sealed end at the bottom of the tar guiding rope 23; the heating wire 26 is wound on the sealed end at the bottom of the tar guiding rope 23 in a noncontact manner, one end of the heating wire 26 is connected with the ejector pin 24 via the conducting wire 27, and the other end thereof is electrically connected with the other electrode of the battery assembly 1 via the other conducting wire 28; the second fiber tube 29 is concentrically arranged at the other side of the heating wire 26 corresponding to the air outlet of the ejector pin 24, forms the airflow channel together with the first fiber tube 25, and is arranged inside the tobacco tar cotton 22 in the vicinity of cigarette holder in order to achieve isolation from tobacco tar. A hollow rubber sheath 32 for sealing and isolation and an atomizer cover 33 that fits the rubber sheath 32 are further arranged inside the cigarette holder-neighboring casing 21 of the atomizer 2, and both the rubber sheath 32 and the atomizer cover 33 are made of a silica gel material.

The disposable electronic cigarette in the present invention is assembled in such a manner that:
1. The curved parts for connection of the heating wire 26 with the conducting wires 27 and 28 are properly welded using a spot welding machine;
2. The heating wire 26 is wound on the U-shaped tar guiding rope 23 in a noncontact manner;
3. The two conducting wires 27 and 28 of the atomizer are respectively welded on the edge of the ejector pin 24 and the screw sleeve 30 using the spot welding machine;
4. The ejector pin 24 is completely wrapped by the tobacco tar cotton 22, and simultaneously, the first fiber tube 25 and the second fiber tube 13 are placed inside the tobacco tar cotton 22 after the ejector pin is wrapped around by the tobacco tar cotton, and the screw sleeve rod with the tobacco tar cotton 22 therein is placed inside the casing 21 of the atomizer;
5. The screw sleeve is pressed into the casing 21 of the atomizer using a die cutting machine.
6. A syringe is completely filled with tobacco tar and then the tobacco tar is injected onto the surface of the tobacco tar cotton 22 through the syringe needle.
7. The grooved rubber sheath 32 is placed inside the casing 21 of the atomizer in parallel and the atomizer cover 33 is pressed into the casing 21 of the atomizer using a punch press.

## Claims

1. A disposable electronic cigarette, comprising a battery assembly (1) and an atomizer (2), **characterized in that**:
the battery assembly (1) comprises,
a cigarette tubular housing (11), one end of the housing (11) being sealed while the other end thereof being provided with an end opening for fitting connection with the atomizer (2), the end opening being provided with positive and negative electrodes capable of electric connection with the atomizer (2), and a cavity with a battery therein being arranged inside the housing (11); and
a battery (12), the battery (12) being arranged inside the cavity of the housing (11), and the positive and negative electrodes of the battery (12) being electrically connected with the positive and negative electrodes at the end opening of the housing;
the atomizer (2) comprises,
a casing (21) having an outer diameter matched with the housing (11) of the battery assembly (1);
cylindrical tobacco tar cotton (22) arranged inside the casing (21), the tobacco tar cotton (22) being of a hollow structure;
a U-shaped tar guiding rope (23) arranged inside the tobacco tar cotton (22), the both sides of the U-shaped tar guiding rope (23) coming into full contact with the tobacco tar cotton (22), and the sealed end at the bottom being arranged in an airflow channel in the hollow tube of the tobacco tar cotton;
a hollow ejector pin (24), one end of the hollow ejector pin being electrically connected with the corresponding electrode of the battery assembly (1) and communicated with an air inlet, while the other end thereof being sleeved with a first fiber tube (25) and arranged inside the hollow tube of the tobacco tar cotton (22), and an air outlet of the ejector pin facing towards the sealed end at the bottom of the tar guiding rope (23);
a heating wire (26), the heating wire (26) being wound on the sealed end at the bottom of the tar guiding rope (23) in a noncontact manner, one end of the heating wire (26) being connected with the ejector pin (24) via a conducting wire (27),and the other end thereof being electrically connected with the other electrode of the battery assembly (1) via the other conducting wire (28); and
a second fiber tube (29), the second fiber tube (29) being concentrically arranged at the other side of the heating wire (26) corresponding to the air outlet of the ejector pin (24), forming the airflow channel together with the first fiber tube (25), and being arranged on the cigarette holder-neighboring end inside the tobacco tar cotton (22), in order to achieve isolation from tobacco tar.

2. The disposable electronic cigarette according to claim 1, **characterized in that** a connection thread (13) is arranged at the end opening where the battery assembly (1) is connected with the atomizer (2), the thread (13) is electrically connected with the negative electrode of the battery, and a battery positive electrode contact is arranged in the center of the end opening;
the atomizer (2) is provided with a conductive screw sleeve (30) that fits the connection thread (13);
one end of the ejector pin (24) is connected with the battery positive electrode contact;
one end of the heating wire (26) is connected with the ejector pin (24) via the conducting wire (27) while the other end thereof is electrically connected with the conductive screw sleeve (30) via the other conducting wire (28).

3. The disposable electronic cigarette according to claim 2, **characterized in that** a rubber cushion (31) is arranged on the inner wall of the conductive screw sleeve (30) and is made of a silica gel material, and the ejector pin (24) is arranged inside the rubber cushion (31).

4. The disposable electronic cigarette according to claim 1, **characterized in that** a hollow rubber sheath (32) for sealing and isolation is further arranged inside the cigarette holder-neighboring casing (21) of the atomizer (2), and the rubber sheath (32) is made of a silica gel material.

5. The disposable electronic cigarette according to claim 4, **characterized in that** an atomizer cover (33) that fits the rubber sheath (32) is arranged inside the cigarette holder-neighboring casing (21) of the atomizer (2).
